Europäisches Patentamt

European Patent Office

Office européen des brevets

(19)

(11) Veröffentlichungsnummer : **0 278 948 B1**

# EUROPÄISCHE PATENTSCHRIFT

(12)

(45) Veröffentlichungstag der Patentschrift :
**15.04.92 Patentblatt 92/16**

(51) Int. Cl.$^5$ : **A61K 35/16**

(21) Anmeldenummer : **88890028.9**

(22) Anmeldetag : **08.02.88**

(54) **Parenteral verabreichbare Blutprodukte.**

(30) Priorität : **12.02.87 AT 299/87**

(43) Veröffentlichungstag der Anmeldung :
**17.08.88 Patentblatt 88/33**

(45) Bekanntmachung des Hinweises auf die
Patenterteilung :
**15.04.92 Patentblatt 92/16**

(84) Benannte Vertragsstaaten :
**BE CH DE ES FR GB IT LI LU NL SE**

(56) Entgegenhaltungen :
**EP-A- 0 120 835**
**WO-A-82/02661**
**DE-A- 2 335 145**
**US-A- 4 465 624**

(73) Patentinhaber : **IMMUNO Aktiengesellschaft**
**Industriestrasse 67**
**A-1221 Wien (AT)**

(72) Erfinder : **Eibl, Martha, Prof.**
**Gustav Tschermakgasse 2**
**A-1180 Wien (AT)**
Erfinder : **Mannhalter, Josef, Dr.**
**Giessaufgasse 27/31**
**A-1050 Wien (AT)**
Erfinder : **Leibl, Heinz, Dr.**
**Kiningergasse 12**
**A-1120 Wien (AT)**

(74) Vertreter : **Wolfram, Gustav, Dipl.-Ing.**
**Schwindgasse 7 P.O. Box 205**
**A-1041 Wien (AT)**

**Beschreibung**

Die Erfindung betrifft parenteral verabreichbare Blutprodukte aus Blutplasma, ein Verfahren zu deren Herstellung sowie deren Verwendung.

Bekannte Verfahren zur Herstellung von menschlichen Blutprodukten beinhalten neben Fraktionierungsmaßnahmen zur Reindarstellung des entsprechenden Produktes auch Verfahren zur Entfernung kontaminierender Substanzen, die Nebenreaktionen hervorrufen können. Beispiele dafür sind Verfahren zur Entfernung vasoaktiver Substanzen (EP 0 120 835, EP 0 122 909) sowie zur Inaktivierung der in solchen Präparationen gegebenenfalls vorkommenden Viren (EP 0 159 311, US-PS 4,297,344).

Bei diesen bekannten Verfahren wurden jedoch die Probleme einer möglichen immunmodulierenden Nebenwirkung der Produkte nicht berücksichtigt. Erste Hinweise für eine mögliche therapiemediierte Immunmodulation geben Studien, die an Hämophiliepatienten durchgeführt wurden. Diese Patienten zeigen eine erhöhte Anfälligkeit gegenüber bestimmten Infektionen bakterieller und viraler Art (vgl. z.B. A.C. Beddall et al., J. Clin. Pathol. 1985 38, 1163-1165; Hämophilie, K. Lechner aus "Handbuch der inneren Medizin", Seite 143, Band II/9, 1985, Springer Verlag; P.H. Levine et al., Health of the Intensively Treated Hemophiliac, With Special Reference to Abnormal Liver Chemistries and Splenomegaly, 1977, 50, 1-9; B.A. McVerry et al., J. Clin. Pathol. 1979 32, 377-381). Weiters weisen Defekte der aus dem peripheren Blut dieser Patienten isolierten Lymphozyten auf Störungen des Immunsystems hin. Als Beispiele hierfür können die Verschiebung des Verhältnisses von T-Helfer- zu T-Suppressorzellen (C.M. Kessler et al. 1983, Lancet, 991-992; P. Saidi et al., 1983, New Engl. J. Med. 308, 1291-1292), die Verringerung der "natural killer"-Zellaktivität (M.M. Lederman et al., N. Engl. J. Med. 1983 308, 79-82) sowie die Erniedrigung der antigenpräsentierenden Kapazität zirkulierender mononuklearer Phagozyten erwähnt werden (J.W. Mannhalter et al., Clin. Immunol. Immunopathol. 1986 38, 390-397).

Die Erfindung löst die geschilderten Probleme durch Bereitstellung eines parenteral verabreichbaren Blutproduktes aus Blutplasma, as frei ist von immunmodulierenden Komponenten, ausgedrückt durch das Fehlen bzw. Ausbleiben einer Niedermodulierung von Fc-Rezeptoren in der Membran von Monozyten, mit den Maßgabe, daß die Expression der Rezeptoren für das Fc-Stück von Immunglobulin G in der Membran der Monozyten nach Interaktion mit der Blutprodukt Präparation nicht oder zu höchstens 30 % herabgesetzt ist.

Darunter wird verstanden, daß die Expression von Rezeptoren für das Fc-Stück von Immunglobulin G in der Membran von Fc-Rezeptor positiven Monozyten nach Interaktion mit einer Blutprodukt-Präparation nicht oder nicht signifikant, d.h. zu höchstens 30 % herabgesetzt ist. Die Expression der Fc-Rezeptoren in der Membran von Monocyten wird durch die Fähigkeit dieser Zellen, mit Immunglobulin G beladene Erythrozyten anzulagern, bestimmt. Die Ergebnisse werden als % Rosetten-formende Zellen (% RFZ) ausgedrückt. Die Bestimmungsmethode der Niedermodulierung der Fc-Rezeptoren ist bekannt und beispielsweise in den Literaturstellen J.W. Mannhalter et al., Clinical Immunology and Immunopathology 1986 38, 390-397; A. Berken et al., J. Exp. Med. 1966 123, 119-144 und C.S. Scott, Clin. exp. Immunol. 1979 38, 300-305 beschrieben.

Die Expression von Rezeptoren für das Fc-Stück von Immunglobulin G (Fc-Rezeptoren) in der Membran von Monozyten ist eine wichtige Voraussetzung für das klaglose Funktionieren der Immunabwehr. Diese Rezeptoren erleichtern nicht nur die Phagozytose opsonisierter Pathogene (Contemporary Topics in Immunobiology, Vol. 14, Regulation of Leukocyte Function, R. Snyderman, ed., Plenum Press New York and London 1971); eine Interaktion dieser Rezeptoren mit ihren Liganden löst auch eine Reihe von Signalen aus, die für den geregelten Verlauf der Immunabwehr von Bedeutung sind ((Induktion der Entstehung von Sauerstoffradikalen (R.B. Jonston et al., J. Exp. Med. 1976 143, 1551-1556; D.N. Rush et al., Cellular Immunology 1984 87, 252-258 und S.D. Wright, J. Exp. Med. 1983 158, 2016-2023), Aktivierung von T-Zellen (J.A. Griffin et al., J. Exp. Med. 1979 150, 653-675 und F.M. Griffin et al., J. Immunol. 1980 125, 844-49), Antigenpräsentation (J.W. Mannhalter et al., Clin. Immunol. Immunopathol. 1986 39, 491-503)). Eine Niedermodulierung der Fc-Rezeptoren hat daher bedeutende klinische Konsequenzen und könnte zu einer Erhöhung der Infektanfälligkeit der mit Blutprodukten behandelten Patienten beitragen.

Die Blutprodukte gemäß der Erfindung, die im wesentlichen frei sind von immunmodulierenden Komponenten, sind erhältlich - durch eine Molekularsiebung der Blutprodukte, u.zw. eine Gelpermeationschromatographie (Gelfiltration) oder

– durch Behandlung der Blutprodukte mit einem molekularsiebartigen Trägermaterial, an dem immunmodulierende Komponenten selektiv adsorbiert werden, oder
– durch Filtration der Blutprodukte über Filter mit einem Porendurchmesser von gleich oder kleiner 10 nm, wobei die Filtration zusätzlich durch Adsorptionswirkung unterstützt werden kann.

Bei der Gelpermeationschromatographie (Gelfiltration) werden die Moleküle nach ihrem Molekulargewicht aufgetrennt. Das Gel besteht aus mikroskopisch kleinen Kügelchen, die Poren einer bestimmten Größe enthalten. Moleküle, die größer als diese Poren sind, können nicht in das Gel eindringen und werden zuerst eluiert. Kleinere Moleküle können in die Gelporen eindringen, werden deshalb zurückgehalten und wandern dadurch langsamer. Auf diese Weise kann in einem Proteingemisch eine Auftrennung der einzelnen Proteine erreicht werden.

Eine bevorzugte Ausführungsform für die selektive Adsorbierung von immunmodulierenden Komponenten besteht darin, daß die Blutprodukte mit einem Adsorptionsmaterial behandelt werden, bestehend aus einem inerten Trägermaterial, wie Sepharose, an dem immobilisierte Liganden, insbesondere Proteine bakteriellen Ursprungs, wie Protein A von Staphylococcen, Protein G bzw. Protein M von Streptococcen gebunden sind.

Ein weiteres Verfahren dieser Art besteht darin, daß die Blutprodukte mit einem Adsorptionsmaterial behandelt werden, bestehend aus einem inerten Trägermaterial, wie Sepharose, an dem Antikörper, die durch Immunisierung mit immunmodulierenden Substanzen gewonnen wurden, gebunden sind.

Bei dem Filtrationsverfahren, welches sich besonders für die Herstellung von Gammaglobulin-Präparationen eignet, werden Teilchen nach ihrer Größe aufgetrennt. Bei dem hier verwendeten Verfahren werden spezielle Filtermembranen mit sehr kleiner Porengröße (10 nm oder kleiner) verwendet. Teilchen, die größer als diese Poren sind, werden im Rahmen des Filtrationsvorganges von der Membran zurückgehalten. Auf diese Weise können aus einem Proteingemisch große (hochmolekulare) Teilchen abgetrennt werden.

Die Blutprodukte gemäß der Erfindung eignen sich mit Vorteil zur Verwendung bei der Behandlung von ererbten und erworbenen Gerinnungsstörungen, zur Behandlung von primären und sekundären Immundefizienzen, sowie zur Behandlung von Autoimmun-, Immunkomplex- und Infektionserkrankungen.

In den folgenden Beispielen sind die Eigenschaften der erfindungsgemäßen Blutprodukte, ihre Herstellung und die Bestimmung einer etwa vorhandenen Niedermodulierung gegenüber Blutprodukt-freien Kontrollmedien näher erläutert.

Beispiel 1:

Es wird eine Faktor VIII-Präparation nach der in der EP 0 127 603 beschriebenen Methode hergestellt. Diese Präparation wurde einer Affinitätschromatographie mit immobilisiertem Protein A nach dem "batch"-Verfahren unterworfen. Dabei wurde eine lyophilisierte Faktor VIII-Präparation, die 500 IE Faktor VIII enthielt, in 20 ml destilliertem Wasser, das mit 10 IE konservierungsmittelfreiem Heparin (Immuno AG, Wien) versetzt war, gelöst und bei Zimmertemperatur über Nacht gegen 2 l eines Citrat-Puffers (0,024 M Natriumcitrat, 0,120 M NaCl, mit HCl auf pH 7,2 eingestellt, versetzt mit 10 IE/ml konservierungsmittelfreiem Heparin, im folgenden kurz "Citrat pH 7,2" genannt) dialysiert. 8 ml des Dialysates (entsprechend etwa 200 IE Faktor VIII) wurden entnommen und mit 2 ml einer Protein A-Sepharose-Suspension (50 %ige Suspension [vol/vol] in Citrat pH 7,2; die Protein A-Sepharose wurde in Citrat pH 7,2 voräquilibriert) vermischt. Danach wurde 4 h bei Zimmertemperatur unter gelegentlichem Schütteln inkubiert. Nach dem Ende der Inkubationszeit wurde die Protein A-Sepharose abzentrifugiert (5 min, 700 x g, Zimmertemperatur), die überstehende Lösung wurde abpippettiert und das Gel noch dreimal mit 10 ml Citrat pH 7,2 gewaschen. Die einzelnen Zentrifugationsüberstände wurden vereinigt, sterilfiltriert, auf 2 IE Faktor VIII/ml eingestellt und bis zur Austestung bei 4°C aufbewahrt.

Die in beschriebener Weise hergestellte und erfindungsgemäß behandelte Faktor VIII-Präparation wurde, wie im folgenden beschrieben, auf ihre immunmodulierende Aktivität geprüft.

Mononukleare Zellen aus peripherem Blut wurden durch Dichtegradientenzentrifugation isoliert (J. W. Mannhalter et al., Clin. Immunol. Immunopathol. 1986 38, 390-397). Dabei wurden 8 ml eines Dichtegradienten (Lymphoprep, Nyegaard & Co., Oslo, Norwegen) mit 20 bis 25 ml heparinisiertem Blut überschichtet und 35 min bei Zimmertemperatur mit 400 xg zentrifugiert. Die mononuklearen Zellen, die sich an der Interphase von Plasma und Gradienten angesammelt hatten, wurden abgesaugt, dreimal mit physiologischer Kochsalzlösung gewaschen und in RPMI-1640-Medium, das 15 % gepooltes, hitzeinaktiviertes (30 min, 56°C) AB-Serum sowie Penicillin (100 IE/ml), Streptomycin (100 myg/ml) und L-Glutamin (2 mM) enthält (RPMI-suppl.), suspendiert.

Die Isolierung der Monozyten aus der mononuklearen Zellpopulation erfolgte durch Adhärenz. Monozyten haben im Gegensatz zu anderen mononuklearen Blutzellen die Eigenschaft, sich an Glas- oder Plastikoberflächen anzulagern. Zur Isolierung der Monozyten werden mononukleare Zellen auf eine Konzentration von 1 x $10^7$ Zellen pro ml RPMI-suppl. eingestellt. 2 ml dieser Suspension werden dann in Plastik-Gewebekulturschalen (Makroplatte TC, Greiner & Söhne, Kremsmünster, Österreich) pipettiert und drei Stunden in einem $CO_2$-Brutschrank (5 % $CO_2$, mehr als 95 % Luftfeuchtigkeit) bei 37°C inkubiert. Dann werden die nicht adhärenten Zellen abpippettiert; die angelagerten Zellen (Monozyten) werden dreimal mit physiologischer Kochsalzlösung gewaschen, mit RPMI-suppl. versetzt und im $CO_2$-Brutschrank bis zur weiteren Verwendung aufbewahrt.

Die Expression von Fc-Rezeptoren in der Membran der Monozyten wurde durch Anlagerung von mit IgG beladenen Rindererythrozyten nachgewiesen (A. Berken et al., J. Exp. Med. 1966 123, 119-144). Rinderblut wurde in Alsever-Lösung abgenommen und mehrmals in phosphatgepufferter Kochsalzlösung, pH 7,2 bis 7,4 (PBS), gewaschen. Dann wurde die Erythrozytenkonzentration auf 2 % in PBS eingestellt. Die Beladung der Erythrozyten mit Immunglobulin G erfolgte durch Inkubation mit einer subagglutinierenden Dosis eines Anti-Rindererythrozyten-Antikörpers (IgG-Fraktion: 0,167 mg/ml PBS, Inkubationszeit eine Stunde, 37°C) (C.S. Scott, Clin. exp. Immunol. 1979 38, 300-305). Nach dem Ende der Inkubationszeit wurde freies IgG durch dreimaliges Waschen in PBS entfernt und die mit Antikörper beladenen Erythrozyten wurden auf eine Konzentration von 0,5 % in PBS, das 0,2 % bovines Serumalbumin (PBS-BSA) enthielt, eingestellt. Diese Erythrozytensuspension konnte nun zum Nachweis der Fc-Rezeptoren in der Monozytenmembran verwendet werden.

Dazu wurden erst die durch Anlagerung an Plastik-Gewebekulturschalen isolierten Monozyten mittels eines Gummiwischers vorsichtig von der Gewebekulturschale abgelöst und auf eine Konzentration von 2 - 3 x $10^6$ Zellen/ml PBS-BSA eingestellt. 100 myl dieser Monozytensuspension wurden dann mit 100 myl der oben beschriebenen Erythrozytensuspension vermischt und bei 4°C während 10 min mit 120 xg zentrifugiert. Nach einer einstündigen Inkubation bei 0°C wurden die pellettierten Zellen vorsichtig resuspendiert und der Prozentsatz der Monozyten, die Erythrozyten angelagert hatten, wurde mit Hilfe eines Phasenkontrastmikroskopes bestimmt. Dabei wurden mindestens 200 Monozyten auf ihre Fähigkeit, mit IgG beladene Erythrozyten anzulagern, untersucht. Die Ergebnisse sind als % Rosetten-formende Zellen (% RFZ) oder als Anlagerungsindex (AI) angegeben. Als Rosette wird ein Monozyt bezeichnet, der mindestens drei Erythrozyten angelagert hat. Mit dem Anlagerungsindex wird die durchschnittliche Anzahl der pro Monozyt angelagerten Erythrozyten angegeben.

Die Prüfung der Faktor VIII-Präparation erfolgte dadurch, daß unmittelbar nach Anlagerung der Monozyten an den Plastik-Gewebekulturschalen die Zellen mit Faktor VIII (2 IE/ml) versetzt und eine Stunde bei 37°C im $CO_2$-Brutschrank inkubiert wurden. Nach Ende der Inkubationszeit wurden die adhärenten Monozyten viermal mit physiologischer Kochsalzlösung gewaschen, mit RPMI-suppl. versetzt und 16 Stunden im $CO_2$-Brutschrank bei 37°C inkubiert. Diese Inkubationszeit ist nötig, damit die in den Blutprodukten enthaltenen Kontaminanten ihre immunmodulierende Wirkung ausüben können. Nach dem Ende dieser 16stündigen Inkubationszeit wurde die Expression der Fc-Rezeptoren in der Monozytenmembran nach der oben beschriebenen Methode bestimmt.

Als Kontrolle wurden Monozyten unmittelbar nach der Anlagerung mit RPMI-1640-Medium, aber ohne Faktor VIII, für eine Stunde inkubiert und im weiteren wie oben beschrieben behandelt.

Die Ergebnisse der Fc-Rezeptor-Expression sind als % Rosetten-formende Zellen (% RFZ) sowie als Anlagerungsindex (AI) angegeben.

---

| Inkubation der Monozyten in Gegenwart von | Faktor VIII-Aktivität | % RFZ[a] | AI[b] |
|---|---|---|---|
| RPMI-Medium (Kontrolle) | - | 81±2 | 4,42±0,05 |
| Faktor VIII (Ausgangsprodukt) | 2 IE/ml | 20±3 | 0,79±0,06 |
| Faktor VIII (erfindungsgemäß mit Protein A-Sepharose behandelt) | 2 IE/ml | 75±4 | 3,78±0,15 |

---

a) % der Monocyten, die drei oder mehr mit IgG beladene Erythrocyten angelagert haben.

b) durchschnittliche Anzahl der angelagerten Erythrozyten pro Monozyt. Für die Errechnung des AI wurden alle gezählten Monozyten (sowohl die, die keine Erythrozyten angelagert haben, als auch alle mit einem oder mehreren angelagerten Erythrozyten) verwendet.

Daraus ist zu ersehen, daß das Faktor VIII-Ausgangsprodukt eine Niedermodulierung der Fc-Rezeptor-Expression von 81 % RFZ auf 20 % RFZ bewirkt. Im Gegensatz dazu beträgt nach Interaktion der Monozyten mit dem erfindungsgemäß behandelten Faktor VIII-Präparat der Prozentsatz der Rosetten-formenden Zellen 75 %. Die Differenz zwischen 81 % RFZ in den Kontrollen und 75 % RFZ nach Interaktion mit der erfindungsgemäß behandelten Faktor VIII-Präparation ist nicht signifikant (Students t-test für paarweise Stichproben). Das bedeutet, daß das erfindungsgemäß behandelte Faktor VIII-Produkt frei von immunmodulierenden Komponenten ist, da, nach Interaktion der Monozyten mit dem erfindungsgemäß behandelten Produkt, sich der RFZ-Wert nicht von dem Kontrollwert (% RFZ bei Monozyten ohne Faktor VIII-Behandlung) unterscheidet.

Beispiel 2:

500 IE einer nach der von Suokela H. et al. in Vox. Sang. 1977 33, 37-50, beschriebenen Methode hergestellten und lyophilisierten Faktor IX-Präparation wurden in 20 ml destilliertem Wasser gelöst und über Nacht gegen 2 l eines Citratpuffers (Citrat pH 7,2, die genaue Zusammensetzung dieses Puffers ist in Beispiel 1 beschrieben) bei 4°C dialysiert. 8 ml (entsprechend etwa 200 IE Faktor IX) wurden entnommen und einer Affinitätschromatographie auf Protein A-Sepharose im Säulenverfahren unterworfen. Dabei wurden die 8 ml Faktor IX-Präparation mit einer Durchflußgeschwindigkeit von 0,1 ml pro min über eine Protein A-Sepharose-Säule (Säule C 10/10 von Pharmacia, 1 ml Gelvolumen), die vorher mit 50 ml Citrat pH 7,2 äquilibriert wurde, gepumpt. Der durch die Säule gepumpte Faktor IX wurde auf 2 IE/ml eingestellt, sterilfiltriert und bis zur Austestung bei 4°C aufbewahrt. Die Prüfung der immunmodulierenden Aktivität der Ausgangspräparation sowie der in der beschriebenen Weise erfindungsgemäß behandelten Faktor IX-Präparation erfolgte, wie in Beispiel 1 dargestellt. Die Ergebnisse dieser Untersuchungen sind in der folgenden Tabelle zusammengefaßt.

| Inkubation der Monozyten in Gegenwart von | Faktor IX-Aktivität | % RFZ[a] | AI[b] |
|---|---|---|---|
| RPMI-Medium (Kontrolle) | - | 75±1 | 4,09±0,04 |
| Faktor IX (Ausgangsprodukt) | 2 IE/ml | 42±3 | 2,31±0,07 |
| Faktor IX erfindungsgemäß be-handelt | 2 IE/ml | 70±3 | 4,00±0,08 |

a) % der Rosetten-formenden Zellen, d.h. % der ausgezählten Monozyten, die drei oder mehr mit IgG beladene Erythrozyten angelagert haben.

b) Anlagerungsindex, d.h. durchschnittliche Anzahl der pro Monozyt angelagerten Erythrozyten.

Aus den Ergebnissen dieser Tabelle ist ersichtlich, daß eine Interaktion von Faktor IX mit Monozyten zu einer Erniedrigung der Fc-Rezeptor-Expression von 75 % RFZ (Kontrollen) auf 42 % RFZ (nach Interaktion mit Faktor IX) führte. Nach Interaktion der Monozyten mit dem erfindungsgemäß behandelten Faktor IX-Produkt betrug die Fc-Rezeptor-Expression jedoch 70 % RFZ. Der Unterschied zwischen 75 % RFZ in den Kontrollen und 70 % RFZ nach Interaktion mit dem erfindungsgemäß behandelten Faktor IX ist nicht signifikant (Students t-test für paarweise Stichproben), und das bedeutet, daß die erfindungsgemäß behandelten Faktor IX-Produkte frei von immunmodulierenden Eigenschaften sind.

Beispiel 3:

Eine lyophilisierte FEIBA-Präparation (500 IE, Immuno AG) wurde in 20 ml destilliertem Wasser gelöst und, wie in den Beispielen 1 und 2 für Faktor VIII bzw. Faktor IX beschrieben, gegen Citrat pH 7,2 dialysiert. Die Entfernung der immunmodulierenden Substanzen erfolgte durch Adsorptionschromatographie mit Protein A-Sepharose nach dem Säulenverfahren. Die genaue Methode war wie in Beispiel 2 für Faktor IX beschrieben. Die Austestung der immunmodulierenden Eigenschaften des Ausgangsproduktes sowie der erfindungsgemäß behandelten FEIBA-Präparation erfolgte, wie in Beispiel 1 beschrieben. Die Ergebnisse dieser Untersuchungen, die in der folgenden Tabelle zusammengefaßt sind, zeigen, daß die erfindungsgemäße Behandlung mit immobilisiertem Protein A die immunmodulierenden Aktivitäten aus FEIBA entfernt.

| Inkubation der Monozyten in Gegenwart von | FEIBA-Aktivität | % RFZ[a] | AI[b] |
|---|---|---|---|
| RPMI-Medium (Kontrolle) | – | 75±1 | 4,09±0,04 |
| FEIBA (Ausgangsprodukt) | 2 IE | 34±4 | 1,89±0,15 |
| FEIBA (nach erfindungsgemäßer Behandlung mit immobilisiertem Protein A) | 2 IE | 73±1 | 3,94±0,05 |

a) % Rosetten-formende Zellen, d.h. Monozyten, die drei oder mehr mit IgG beladene Erythrozyten angelagert haben.

b) Anlagerungsindex, d.h. durchschnittliche Anzahl der pro Monozyt angelagerten Erythrozyten.

Es ist festzustellen, daß bei allen untersuchten Gerinnungsfaktor-Präparaten (Faktor VIII, Faktor IX, FEIBA) die immunmodulierenden Eigenschaften durch Behandlung mit immobilisiertem Protein A sowohl nach dem "batch"-Verfahren als auch nach dem Säulenverfahren entfernt werden.

Beispiel 4:

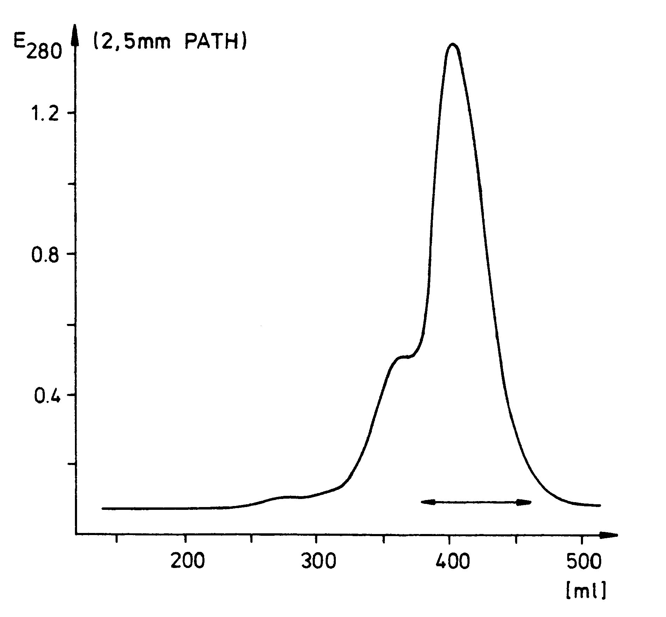

Eine für intramuskuläre Anwendung geeignete Immunglobulin-G-Präparation (i.m. IgG) wurde durch Äthanol-Fraktionierung (H.F. Deutsch et al., J. Biol. Chem. 1946 164, 109ff.) hergestellt. Diese Präparation wurde einer Gelpermeationschromatographie unterworfen. Dabei wurde eine Trennsäule (K 50/60, Pharmacia) mit 750 ml Chromatographiegel (Sephacryl S 300, Pharmacia) befüllt und mit einem Phosphatpuffer (20 mM $KH_2PO_4$, 0,15 M NaCl, 0,02 % $NaN_3$, pH 8,0) äquilibriert. Diese Säule wurde dann mit 300 mg i.m. IgG (in 10 ml Phosphatpuffer) beladen und bei einer Durchflußgeschwindigkeit von 1 ml pro min eluiert. Die Eluate wurden in 11 ml Fraktionen aufgefangen und der Proteingehalt wurde mittels eines UV-Detektors bei 280 nm bestimmt. Dabei ergaben sich Elutionskurven, wie sie in der Zeichnung dargestellt sind. Die mit ↔ bezeichneten Fraktionen wurden vereinigt und auf immunmodulierende Aktivität, wie in Beispiel 1 beschrieben, geprüft. Die Ergebnisse dieser Untersuchungen sind der folgenden Tabelle zu entnehmen, woraus ersichtlich ist, daß diese Behandlung ausgereicht hat, um die Immunglobulin-G-Präparation frei von immunmodulierenden Komponenten zu machen. Der RFZ-Wert, der nach Interkation der Monozyten mit dem erfindungsgemäß behandelten i.m. IgG erhalten wurde, stimmt im wesentlichen mit dem Kontrollwert überein.

7

| Inkubation der Monozyten in Gegenwart von | i.m. IgG mg/ml | % RFZ[a] | AI[b] |
|---|---|---|---|
| RPMI-Medium (Kontrolle) | - | $84\pm3$ | $7{,}11\pm0{,}22$ |
| i.m. IgG (Ausgangsmaterial) | 10,8 | $20\pm2$ | $1{,}97\pm0{,}07$ |
| i.m. IgG (nach erfindungs- gemäßer Behandlung durch Gelpermeationschromato- graphie) | 11,3 | $64\pm2$ | $5{,}94\pm0{,}04$ |

a) % Rosetten-formende Zellen, d.h. Monozyten, die drei oder mehr mit IgG beladene Erythrozyten angelagert haben.

b) Anlagerungsindex, d.h. durchschnittliche Anzahl der pro Monozyt angelagerten Erythrozyten.

Beispiel 5:

Eine, wie im Beispiel 4 beschrieben, hergestellte Immunglobulin-G-Präparation (i.m. IgG) wurde auf eine Konzentration von 50 mg/ml in phosphatgepufferter Kochsalzlösung (PBS) eingestellt. Nach entsprechender Vorklärung über geeignete Filter wurde das Präparat durch ein spezielles Filter mit sehr kleiner Porengröße (Sartorius SM 11318, Porengröße 10 nm) geschickt. Dabei wurde die Filtermembran in einen geeigneten Filterhalter eingelegt und z.B. 20 ml der i.m. IgG-Lösung mit einem Druck von 1 bis 1,5 bar durch das Filter geschickt. Das Filtrat wurde dann, wie im Beispiel 1 beschrieben, auf etwaige immunmodulierende Eigenschaften untersucht. Die in der folgenden Tabelle angegebenen Ergebnisse zeigen, daß diese Behandlung den Großteil der immunmodulierenden Eigenschaften des Präparates entfernt. Während eine Behandlung der Monozyten mit dem i.m. IgG-Ausgangsprodukt eine Niedermodulierung der Fc-Rezeptor-Expression von 79 % auf 21 % bewirkt, beträgt die Fc-Rezeptor-Expression der Monozyten nach Interaktion mit dem erfindungsgemäß behandelten Produkt 67 %. Die erfindungsgemäß behandelte i.m. IgG-Fraktion ist also im wesentlichen frei von immunmodulierenden Eigenschaften.

| Inkubation der Monozyten in Gegenwart von | i.m. IgG mg/ml | % RFZ[a] |
|---|---|---|
| RPMI-Medium (Kontrolle) | – | $79 \pm 3$ |
| i.m. IgG (Ausgangsmaterial) | 10,0 | $21 \pm 3$ |
| i.m. IgG (nach erfindungs- gemäßer Behandlung durch Filtration durch spezielle Filter mit 10 nm Porengröße) | 10,0 | $67 \pm 2$ |

a) % Rosetten-formende Zellen, d.h. Monozyten, die drei oder mehr mit IgG beladene Erythrozyten angelagert haben.

Beispiel 6:

Es wurde eine Faktor VIII-Präparation nach der in der EP-A- 0 127 603 beschriebenen Methode hergestellt. Diese Präparation wurde einer Affinitätschromatographie mit immobilisierten, gegen die immunmodulierende Komponente gerichteten Antikörpern unterworfen.

Um die für die Affinitätschromatographie benötigten Antikörper herzustellen, wurde zuerst die immunmodulierende Komponente aus einer wie oben beschrieben hergestellten Faktor VIII-Präparation isoliert. Dies wurde mit einer Kombination aus Affinitätschromatographie und Säulenchromatographie erreicht. Dazu wurden 2500 IE von Faktor VIII in 100 ml destilliertem Wasser gelöst und für 24 Stunden gegen Citrat pH 7,2 dialysiert. Dieses Produkt wurde dann auf eine Säule, die mit immobilisiertem Protein A (Protein A-Sepharose, 12 ml Gelvolumen, Säule C 10/20, Pharmacia, Schweden) befüllt war, aufgetragen und über Nacht bei Zimmertemperatur und einer Durchflußgeschwindigkeit von 0,5 ml/min zirkuliert. Anschließend wurde die Säule mit 100 ml Citrat pH 7,2 (Durchflußrate 0,5 ml/min) gewaschen. Das an die Protein A-Sepharose gebundene Material wurde anschließend mit einem sauren Citratpuffer (50 mM Trinatriumcitrat, 50 mM Zitronensäure, pH 3,0; im folgenden kurz "Citrat pH 3,0" genannt) eluiert und sofort nach der Elution mit 1 M Natriumphosphatpuffer, pH 8,5, neutralisiert. Nach Dialyse gegen PBS wurde dieses Material einer Molekularsiebchromatographie unterworfen. Dabei wurde das dialysierte Protein A-Eluat auf eine Superase Prep Grade Säule (HR 16/50) aufgetragen und mit einer Durchflußgeschwindigkeit von 0,5 ml/min chromatographiert. Die Fraktionen mit einem Molekulargewicht von größer 400 kD enthielten die immunmodulierende Komponente, wie durch den bereits beschriebenen Rosettentest festgestellt wurde. Diese Fraktionen wurden gepoolt und zur Herstellung von Antikörpern in Versuchstieren verwendet.

Es wurden 150 myg der immunmodulierenden Komponente (in 200 myl PBS) mit 200 myl komplettem Freund'schen Adjuvans vermischt und damit ein Kaninchen auf zwei Stellen subkutan immunisiert. Am 21. und 28. Tag nach der Erstimmunisierung wurde auf die gleiche Art und Weise eine Booster-Immunisierung durchgeführt. Ab dem 35. Tag wurden sechs Wochen lang in wöchentlichem Abstand etwa 30 ml Blut abgenommen und daraus Serum hergestellt. Anschließend wurden die Serumproben der verschiedenen Blutabnahmedaten vereinigt und daraus die Immunglobulin-G-Fraktion isoliert.

Dabei wurde das Serum mit Ammoniumsulfat bis zu einer 35 %igen Sättigung versetzt, 25 Minuten bei Raumtemperatur gerührt und anschließend bei 800 x g 15 Minuten lang zentrifugiert. Der Niederschlag wurde in zu 33 % gesättigtem Ammoniumsulfat resuspendiert, 15 Minuten gerührt und erneut abzentrifugiert. Der dabei erhaltene Niederschlag wurde in PBS, das 0,1 % Natriumazid enthielt, gelöst und einer Molekularsiebchromatographie mittels einer Superase Prep Grade Säule (HR 16/50, Durchflußrate 0,5 ml/min) unterzogen. Die Fraktionen im Molekulargewichtsbereich von 150 kD (also die Immunglobulin-G enthaltenden Fraktionen) wurden gesammelt und gegen 0,1 M Natriumhydrogencarbonat, 0,5 M Natriumchlorid, pH 8,5 (= Kopplungspuffer) dialyisiert.

Die so gewonnenen Antikörper wurden dann durch Kopplung an Cyanogenbromid(CNBr)-aktivierte Sepharose immobilisiert (Vorschrift der Firma Pharmacia). Dabei wurden 4 g CNBr-aktivierte Sepharose 4B (Firma Pharmacia, Schweden) in 1 mM Salzsäure gequollen und auf einer Glasfilternutsche mit 500 ml 1 mM Salzsäure gewaschen. 10 ml des Gels wurden mit 40 ml des im Kopplungspuffer befindlichen Antikörpers (entsprechend 90 mg Protein) gemischt und unter gelegentlichem Schütteln 2 Stunden bei Raumtemperatur inkubiert. Dann wurde abzentrifugiert (800 x g, 15 min), durch Resuspendieren in Kopplungspuffer und anschließende Zentrifugation gewaschen und das Gelmaterial in 50 ml Blockierungspuffer (= Kopplungspuffer + 1 M Äthanolamin) suspendiert. Nach einer Inkubation von 2 Stunden bei Raumtemperatur wurde das Gelmaterial gewaschen, u.zw. zuerst in 100 ml 0,1 M Tris, 0,5 M Natriumchlorid, pH 8,0 und zweitens in 100 ml 0,1 M Eisessig, 0,5 M Natriumchlorid, pH 4,0. Die pH 8 - pH 4-Waschschritte wurden dreimal wiederholt. (Das Gelwaschen wurde durch Resuspendieren und Abzentrifugieren durchgeführt.) Zuletzt wurde das Gelmaterial in 20 ml PBS + 10 IE Heparin/ml suspendiert. Eine Analyse zeigte, daß 9 mg des gegen die immunmodulierende Komponente gerichteten Antikörpers pro ml Gel gebunden waren.

4,5 ml dieses Gels wurden in eine Chromatographiesäule (C 10/10, Pharmacia, Schweden) gepackt und der Reihe nach mit 30 ml 3 %igem bovinem Serumalbumin (in PBS), mit 50 ml 50 mM Trinatriumcitrat, mit 50 mM Zitronensäure (pH 3,0) und schließlich mit 200 ml PBS (enthaltend 10 IE/ml Heparin) gespült (Durchflußrate 0,5 ml/min). Dieses Gelmaterial wurde dann zur Abtrennung der immunmodulierenden Komponente verwendet.

Diese Abtrennung kann sowohl im "batch"- als auch im Säulenverfahren durchgeführt werden. Im Beispiel wird die Abtrennung der immunmodulierenden Komponente aus einer Faktor VIII-Präparation, durchgeführt im Säulenverfahren, beschrieben. Nach demselben Prinzip kann aber auch die immunmodulierende Komponente aus Faktor IX- und FEIBA-Präparationen entfernt werden.

Im Beispiel wurden 500 IE eines wie oben beschrieben hergestellten Faktor VIII-Präparates in 20 ml destilliertem Wasser gelöst und zweimal 24 Stunden gegen je einen Liter PBS, das 10 IE/ml Heparin enthielt, dialysiert. Danach wurde die Konzentration des Faktor VIII-Präparates mit demselben Puffer auf 10 IE Faktor VIII pro ml eingestellt. 100 IE dieses Materials wurden dann auf die oben beschriebene Trennsäule (Sepharose, an die Antikörper gegen die immunmodulierende Komponente gekoppelt worden waren) aufgetragen und 16 Stunden bei Raumtemperatur mit einer Durchflußgeschwindigkeit von 0,5 ml/min über die Säule zirkuliert. Das nicht an die Säule gebundene Material wurde dann auf 2 IE Faktor VIII/ml eingestellt und, wie im Beispiel 1 beschrieben, mittels des Rosettentests auf die immunmodulierende Aktivität getestet. Die Ergebnisse dieser Untersuchungen sind in der folgenden Tabelle zusammengefaßt.

| Inkubation der Monozyten in Gegenwart von | Faktor VIII-Aktivität | % RFZ[a] | AI[b] |
|---|---|---|---|
| RPMI-Medium (Kontrolle) | – | 81±3 | 6,06±1,76 |
| Faktor VIII (Ausgangsprodukt) | 2 IE/ml | 31±5 | 1,36±0,13 |
| Faktor VII (erfindungsgemäß behandelt) | 2 IE/ml | 80±6 | 4,79±1,45 |

a) % der Rosetten-formenden Zellen, d.h. % der ausgezählten Monozyten, die drei oder mehr mit IgG beladene Erythrocyten angelagert haben.

b) Anlagerungsindex, d.h. durchschnittliche Anzahl der pro Monozyt angelagerten Erythrozyten.

Die Ergebnisse, die in dieser Tabelle dargestellt sind, zeigen deutlich eine immunmodulierende Aktivität des Faktor VIII-Ausgangsproduktes. Eine Interaktion der Monozyten mit dem Ausgangsprodukt führte zu einer Erniedrigung der Fc-Rezeptor-Expression von 81 ± 3 % RFZ (Kontrollen) auf 31 ± 5 % RFZ (nach Interaktion mit Faktor VIII). Im Gegensatz dazu führte eine Interaktion der Monozyten mit dem erfindungsgemäß behandelten Faktor VIII-Produkt zu keiner Reduktion von Fc-Rezeptoren in der Monozytenmembran (81 ± 3 % RFZ nach Interaktion mit Kontrollmedium gegenüber 80 ± 6 % RFZ nach Interaktion mit dem erfindungsgemäß behandelten Faktor VIII-Produkt). Der Unterschied zwischen 81 ± 3 % RFZ und 80 ± 6 % RFZ ist statistisch nicht signifikant (Students t-Test für paarweise Stichproben); und das bedeutet, daß das erfindungsgemäß behandelte Faktor VIII-Produkt frei von immunmodulierenden Eigenschaften ist.

**Patentansprüche**

1. Parenteral verabreichbares Blutprodukt aus Blutplasma, das frei ist von immunodulierenden Komponenten, ausgedrückt durch das Fehlen bzw. Ausbleiben einer Niedermodulierung von Fc-Rezeptoren in der Membran von Monozyten, mit der Maßgabe, daß die Expression der Rezeptoren für das Fc-Stück von Immunglobulin G in der Membran der Monozyten nach Interaktion mit der Blutprodukt-Präparation nicht oder zu höchstens 30 % herabgesetzt ist.

2. Parenteral verabreichbares Blutprodukt aus Blutplasma nach Anspruch 1, erhältlich
– durch eine Molekularsiebung des Blutproduktes, u.zw. durch Gelpermeationschromatographie (Gelfiltration) oder
– durch Behandlung des Blutproduktes mit einem molekularsiebartigen Trägermaterial, an dem immunmodulierende Komponenten selektiv adsorbiert werden, oder
– durch Filtration des Blutproduktes über Filter mit einem Porendurchmesser von gleich oder kleiner 10 nm.

3. Verfahren zur Hertellung von Blutprodukten nach Anspruch 1 oder 2, dadurch gekennzeichnet, daß die Blutprodukte mit einem Adsorptionsmaterial behandelt werden, bestehend aus einem inerten Trägermaterial, wie Sepharose, an dem immobilisierte Liganden, insbesondere Proteine bakteriellen Ursprungs, wie Protein A von Staphylococcen, Protein G bzw. Protein M von Streptococcen gebunden sind.

4. Verfahren zur Herstellung von Blutprodukten nach Anspruch 1 oder 2, dadurch gekennzeichnet, daß die Blutprodukte mit einem Adsorptionsmaterial behandelt werden, bestehend aus einem inerten Trägermaterial, wie Sepharose, an dem Antikörper, die durch Immunisierung mit immunmodulierenden Substanzen gewonnen wurden, gebunden sind.

5. Verwendung von Blutprodukten nach den Ansprüchen 1 oder 2 bei der Herstellung von Präparationen zur Behandlung von ererbten und erworbenen Gerinnungsstörungen, zur Behandlung von primären und sekundären Immundefizienzen, sowie zur Behandlung von Autoimmun-, Immunkomplex- und Infektionserkrankungen.

## Claims

1. Blood product made from blood plasma for parenteral administration, which is devoid of immune-modulating components, expressed by the lack or absence of a down-modulation of Fc receptors in the membrane of monocytes, with the proviso that the expression of the receptors for the Fc fragment of immunoglobulin G in the membrane of the monocytes, after interaction with the blood product preparation, is not reduced or is reduced by maximumly 30 %.

2. Blood product made from blood plasma for parenteral administration according to claim 1, obtainable
   – by a molecular sieving of the blood product, namely by gel permeation chromatography (gel filtration) or
   – by treatment of the blood product with a molecular-sieve-like carrier material, to which immune-modulating components are adsorbed selectively, or
   – by filtration of the blood product over filter means having a pore diameter of equal to or smaller than 10 nm.

3. Method of producing blood products according to claim 1 or 2, characterised in that the blood products are treated with an adsorptive material consisting of an inert carrier material, such as Sepharose, to which immobilised ligands, in particular proteins of bacterial origin, such as protein A of Staphylococci, protein G or protein M of Streptococci, are bound.

4. Method of producing blood products according to claim 1 or 2, characterised in that the blood products are treated with an adsorptive material consisting of an inert carrier material, such as Sepharose, to which antibodies obtained by immunization with immune-modulating substances are bound.

5. Use of blood products according to claim 1 or 2 in the production of preparations for treating inherited or acquired coagulation disorders, for the treatment of primary and secondary immunodeficiencies, as well as for the treatment of autoimmune, immune-complex and infectious diseases.

## Revendications

1. Produit sanguin administrable par voie parentérale, provenant de plasma sanguin, qui est exempt de composants immunomodulateurs, exemption exprimée par le défaut ou l'absence d'une hypomodulation des récepteurs Fc dans la membrane de monocyte dans la mesure où l'expression des récepteurs pour le fragment Fc de l'immunoglobuline G dans la membrane des monocytes n'est pas abaissée ou que d'un maximum de 30 % après interaction avec la préparation de produit sanguin.

2. Produit sanguin administrable par voie parentérale, provenant de plasma sanguin, selon la revendication 1, qui peut être obtenu
   – par tamisage moléculaire du produit sanguin, à savoir par chromatographie par perméation du gel (filtration sur gel) ou
   – par traitement du produit sanguin par un matériau support de type tamis moléculaire sur lequel sont adsorbés sélectivement les composants immunomodulateurs, ou
   – par filtration du produit sanguin sur un filtre à pores de diamètre inférieur ou égal à 10 nm.

3. Procédé de préparation de produits sanguins selon la revendication 1 ou 2, caractérisé en ce que les produits sanguins sont traités par un matériau adsorbant composé d'un matériau support inerte tel que le sépharose, auquel sont liés des ligands immobilisés, notamment des protéines d'origine bactérienne, telle que la protéine A de staphylocoques, la protéine G ou la protéine M de streptocoques.

4. Procédé de préparation de produits sanguins selon la revendication 1 ou 2, caractérisé en ce que les produits sanguins sont traités par un matériau adsorbant composé d'un matériau support inerte tel que le sépharose auquel sont liés des anticorps obtenus par immunisation par des substances immunomodulatrices.

5. Emploi de produits sanguins selon la revendication 1 ou 2 lors de la fabrication de préparations destinées au traitement des troubles héréditaires ou acquis de la coagulation, au traitement des déficits immunitaires primaires et secondaires, ainsi qu'au traitement des maladies auto-immunes, des maladies des complexes immuns et des infections.